# EUROPEAN PATENT SPECIFICATION

(11) **EP 1 222 468 B1**
(45) Date of publication and mention of the grant of the patent: **31.03.2004**
(21) Application number: 00970956.9
(22) Date of filing: 13.10.2000
(51) Int. Cl.: G01N 33/68, G01N 33/543

(54) **MULTIPLEX CYTOKINE ANALYSIS**
MULTIPLE ANALYSE VON ZYTOKINEN
ANALYSE DE CYTOKINE MULTIPLEX

(30) Priority: 13.10.1999 US 417292
(43) Date of publication of application: 17.07.2002
(73) Proprietor: INCYTE CORPORATION, Palo Alto, California 94304 (US)
(72) Inventor: JOHANN, Timothy, Palo Alto, CA 94304 (US); IRAO, Sheila, Palo Alto, CA 94304 (US)
(74) Representative: Wright, Simon Mark
(86) International application number: PCT/US2000/028635
(87) International publication number: WO 2001/027611

(56) References cited:
- WO-A-99/40434
- US-A- 5 804 370
- CARSON R T ET AL: "Simultaneous quantitation of 15 cytokines using a multiplexed flow cytometric assay" JOURNAL OF IMMUNOLOGICAL METHODS,NL,ELSEVIER SCIENCE PUBLISHERS B.V.,AMSTERDAM, vol. 227, no. 1-2, 30 July 1999 (1999-07-30), pages 41-52, XP004178041 ISSN: 0022-1759
- MAO JENNY T ET AL: "Cocaine down-regulates IL-2-induced peripheral blood lymphocyte IL-8 and IFN-gamma production." CELLULAR IMMUNOLOGY, vol. 172, no. 2, 1996, pages 217-223, XP002164564 ISSN: 0008-8749

## Description

The field of the invention is cytokine analysis.

### Background

Cytokines are signaling proteins which are important in many diverse fields of research and in the diagnosis and clinical treatment of disease. For example, cytokines regulate the body's inflammatory response to infection. Tumor necrosis factor alpha, interleukin-1, interleukin-6, interleukin-8, and interleukin-12 are pro-inflammatory cytokines which have been implicated with sepsis. Anti-inflammatory cytokines such as interleukin-4 and interleukin-10 are also produced in response to infection to turn down the inflammatory response. Monitoring the serum levels of these and other cytokines can provide a measure of immune activity in the host. For example, several clinical studies have demonstrated a statistical link between TNF-a levels and severity of infection. Furthermore, research has indicated that different pathogens elicit unique patterns of cytokine expression. These data demonstrate that cytokines are excellent molecular markers for the diagnosis of septic inflammatory response.

The current methods of choice for the analysis of cytokine expression in blood samples are quantitative RT-PCR and ELISA. Quantitative RT-PCR measures the level of cytokine mRNA in cells interest. This method requires the extraction ofmRNA from cells and thus requires a great deal of pre-assay preparation. The rate determining step of this assay is also the capital intensive step (requiring the expensive RT-PCR instrument) which severely limits scaling up the throughput of this method. Currently, only two cytokines can be detected per assay reaction with quantitative RT-PCR which also limits the potential throughput of this assay. ELISA, on the other hand, directly measures the concentration of cytokine protein in serum or cell supernatants. ELISA assays require no pre-assay preparation. The capital intensive step (detection) is not the longest step in ELISA which allows greater potential throughput than quantitative RT-PCR. These assays do, however, only measure the concentration of one cytokine per reaction which limits their throughput. R. T. Carson & D. A. A. Vignali (1999), J. Immunol. Meth. 227, 41-52 discloses a multiplexed flow cytometry-based assay for simultaneous quantification of 15 cytokines, in which capture antibodies, each one being specific for a given cytokine, are immobilized on beads. Bead sets comprising beads coated with different antibodies are placed in an array of sealed tubes.

The present invention relates to antibody array technology - a powerful tool for the parallel analysis of multiple gene products at the protein level. This technology complements that of DNA microarrays by facilitating the analysis of post transcriptional regulatory events. More particularly, the invention provides ELISA-type assays which analyze the concentrations of multiple proteins such as cytokines per reaction.

### SUMMARY OF THE INVENTION

The invention provides methods and compositions for the simultaneous, quantitative detection of cytokines in a sample. The methods use a solid phase array comprising a plurality of different antibodies arrayed in corresponding discrete array elements and specific for a corresponding plurality of different cytokines. In particular embodiments, the cytokines are selected from GM-CSF, IL1α, IL1β, IL2, IL4, IL6, IL7, IL8, IL10, IL12, TNFα, APO-1, sICAM-1, IFN-α, IFN-γ, IL3, IL5, IL13, IL15, IL16, MCP-1, SAA and sVCAM-1; the plurality is at least five, preferably at least eight, more preferably eleven; the antibodies are monoclonal; each antibody is bound to a different one of the corresponding cytokines; and the array is a microarray on a plastic or glass substrate made by contact deposition. The invention also provides methods of manufacturing and using the arrays for the simultaneous, quantitative detection of cytokines in samples.

### DESCRIPTION OF SPECIFIC EMBODIMENTS OF THE INVENTION

The following descriptions of particular embodiments and examples are offered by way of illustration and not by way of limitation. Unless contraindicated or noted otherwise, in these descriptions and throughout this specification, the terms "a" and "an" mean one or more and the term "or" means and/or.

The methods use solid phase arrays of cytokine-specific antibodies arrayed in corresponding discrete array elements. The array elements are discrete regions of a substrate surface in fluid connection such that all the elements of the array can be incubated, washed, etc. in a single continuous medium. Hence, an array is distinct from assay formats where each specific antibody is separated in discrete, fluid-separated incubation wells as in a microtiter plate.

The antibody arrays may be constructed by a number of methods known in the art on a wide variety of substrates such as glass, silicon, plastics, nylon membranes, etc., including contact deposition, e.g. US Pat Nos. 5,807,522; 5,770,151, etc.; flow path-based methods, e.g. US Pat No. 5,384,261; dip-pen nanolithography-based methods, e.g. Piner, et al., Science Jan 29 1999: 661-663, etc.; etc. See also copending Serial No. 09/150,502, describing capillary printing systems which may be used for antibody array manufacturing. In a preferred embodiment, the antibodies are arrayed at corresponding discrete elements in high density, i.e. microarrays, generally at least 100, preferably at least 1000, more preferably at least 10,000, most preferably at least 100,000 discrete elements per square centimeter.

The antibodies may be intact or fragments, purified or recombinantly expressed, monoclonal or polyclonal and may be affinity purified. In a particular embodiment, each cytokine-specific antibody of the array is a monoclonal antibody.

The antibodies may be specific for pluralities of a wide variety of cytokines, particularly lymphokines. Suitable cytokines may be purchased in purified or recombinant from commercial sources, expressed from commercially and/or publically available clones, and/or purified from tissues. In a particular embodiment, the cytokines are of native human sequence, though homologs from a wide variety of animal species, particularly mammalian (e.g. murine) species are frequently available and may be used. One unexpected finding of the invention was that a plurality of different cytokines could be simultaneously measured in the same, single incubation, particularly where the plurality is at least five, more particularly at least eight, most particularly at least eleven different cytokines. Exemplary human sequence cytokines shown to be so detectable are shown in Table 1.

**Table 1.**

| Cytokines shown to be specifically, quantitatively detectable in muliplex immunoassays. | | | | | |
|---|---|---|---|---|---|
| GM-CSF | IL4 | IL10 | sICAM-1¹ | IL5 | MCP-1² |
| IL1α | IL6 | IL12 | IFN-α | IL13 | SAA³ |
| IL1β | IL7 | TNFα | IFN-γ | IL15 | sVCAM-1⁴ |
| IL2 | IL8 | APO-1 | IL3 | IL16 | |

| | | | | | |
|---|---|---|---|---|---|
| ¹ Intercellular Adhesion Molecule | | | | | |
| ² Macrophage Chemoattractant Protein | | | | | |
| ³ Serum Amyloid A | | | | | |
| ⁴ Soluble Vascular Cell Adhesion Molecule | | | | | |

Accordingly, the disclosed arrays may be used to simultaneously, quantitatively detect such cytokines in samples. A wide variety of cytokine containing samples maybe subject to analysis by these methods, e.g. serum, urine, cerebral spinal fluid, etc. Furthermore, unlike PCR-based methods, antibody array technology can measure gene expression in samples which contain no RNA. This greater availability results in the ability to conduct detailed statistical studies with antibodies arrays which are not possible with other assays.

Incubation and wash conditions are readily determined empirically, as shown below, such that the targeted plurality of cytokines may be simultaneously assayed in a single incubation medium. A wide variety of methods may be used to detect the specifically bound cytokine analytes, including directly labeled cytokine specific antibody reagents, sandwich format immunoassays, cytokine-receptor binding assays, etc. Table 2 shows various cytokine pluralities simultaneously assayed by exemplary methods.

**Table 2.**

| Specific, Quantitative Detection of Cytokines in Micro-Array Immunoassays | | | | | |
|---|---|---|---|---|---|
| Exp | Antibody | Specificity | Array | Detection Format | Specific, Quantitative Detection |
| #12 | Mabs | IL1α, IL1β, IL2, IL4, IL12 | polystryene, low density, contact depostion | sandwich assay, HRP-STR | +, +, +, +, +, + |
| #16 | Affinity purified polyclonals | IL1α, IL1β, IL2, IL4, IL6, IL7, IL8, IL10, IL12 | glass, high density, contact depostion | ³H-labeled cytokine-specific antibody | +, +, +, +, +, +, +, +, + |
| #25 | Mabs | GM-CSF, IL1α, IL1β, IL2, IL4, IL6, IL7, IL8, IL10, IL12, TNFα | glass, high density, contact depostion | sandwich assay, HRP-STR | +, +, +, +, +, +, +, +, +, +, + |
| #27 | Mabs | GM-CSF, IL1α, IL1β, IL2, IL4, IL6, IL7, IL8, IL10, IL12, TNFα | nylon, high density, ink jet deposition | sandwich assay, rhodamine conjugate | +, +, +, +, +, +, +, +, +, +, + |
| #33 | Mabs | GM-CSF, IL1α, IL1β, IL2, IL4, IL6, IL7, IL8, IL10, IL12, TNFα | polystyrene, high density, contact depostion | sandwich assay, HRP-STR | +, +, +, +, +, +, +, +, +, +, + |
| #51 | Mabs | APO-1, sICAM-1, IFN-α, IFN-γ, IL3, IL5, IL13, IL15, IL16, MCP-1, SAA, sVCAM-1 | glass, high density, contact depostion | sandwich assay, HRP-STR | +, +, +, +, +, +, +, +, +, +, +, + |
| #55 | Mabs | GM-CSF, TNFα, APO-1, sICAM-1, IFN-α, IFN-γ, MCP-1, SAA, sVCAM-1 | glass, high density, contact depostion | sandwich assay, HRP-STR | +, +, +, +, +, +, +, +, + |
| #62 | Mabs | IL1α, IL1β, IL2, IL4, IL6, IL7, IL8, IL10, IL12, IL3, IL5, IL13, IL15, IL16. | glass, high density, contact depostion | sandwich assay, HRP-STR | +, +, +, +, +, +, +, +, +, +, +, +, +, + |
| #86 | Mabs | Table 1 cytokines | polystyrene, high density, contact depostion | sandwich assay, HRP-STR | +, +, +, +, +, +, +, +, +, +, +, +, +, +, +, +, +, +, +, +, +, +, + |

### EXEMPLARY EXPERIMENTAL PROTOCOLS

Using the following protocols, we constructed a prototype antibody array for the simultaneous detection of 11 different cytokines in serum and cell supernatants. This assay was based on an ELISA sandwich format with chemiluminescent detection.
Chemiluminescent detection, as opposed to fluorescent detection, requires no excitation of sample and allows the simultaneous detection of several different arrays at once resulting in a substantial increase in the throughput. Eleven distinct cytokines were successfully simultaneously, quantitatively assayed and the measurements validated by comparison with RT-PCR assays.

Array construction. Capture antibodies (Biosource International) for 11 cytokines (GM-CSF, IL1α, IL1β, IL2, IL4, IL6, IL7, IL8, IL10, IL 12 and TNFα) were spotted at a concentration of 0.5 mg/ml directly into the bottom of 12 well plates (Corning) which had been pre-coated for optimal antibody binding. Biotinylated bovine albumin was spotted at a concentration of 100 ug/ml as fiduciary material. Array elements were roughly 1000 microns in diameter with 3000 micron center to center spacing. Once spotted, plates were immediately covered and incubated overnight at 4°C. Arrays were then washed 4x with a solution ofPBS (10 mM phosphate, 2.7 mM potassium chloride, 137 mM sodium chloride, pH 7.4) and 0.1% Tween-20 (Sigma) to remove unbound antibody. Plates were then blocked for 2 hours at room temperature with a solution containing 150 mM sodium, 4 mM potassium, 140 mM chloride, 10 mM phosphate (pH 7.4) and 5% BSA.

Assay conditions. The BSA solution was removed from each array followed by 4 washes with a solution of 0.005% tween-20 in PBS. 1 ml of sample was then applied to each array and incubated with shaking (120 rpm) for 2 hours at room temperature. Samples were removed and arrays washed 4x with 0.005% tween-20 in PBS. 1 ml of a solution containing biotinylated antibodies to each of the 11 cytokines (GM-CSF, IL1α, IL1β, IL2, IL4, IL6. IL7, IL8, IL10, IL12 and TNFα) at a concentration of 0.5 ug/ml in 150 mM sodium, 4 mM potassium, 140 mM chloride, 10 mM phosphate (pH 7.4) and 10% BSA was added to each well and incubated with shaking at room temperature for 1 hour. The antibody solution was removed and 1 ml of a horseradish-peroxidase-streptavidin conjugate (HRP-STR), 0.2 ug/ml in HPE-dilution buffer (Research Diagnostics Inc.) was added to each array and incubated for 30 minutes at room temperature with shaking. The HRP-STR solution was removed and arrays washed 4X with 0.005% Tween-20 in PBS solution. SuperSignal™ (Pierce) chemiluminescent substrate was mixed 1:1 with provided peroxide solution, 500 ul of this mixture was added to each array, and the entire plate imaged simultaneously with a peltier cooled CCD camera imaging system (ChemiImager™ 4000, Alpha Innotech Co.). X-Ray film can also be used as a detection system. Data can be quantitated with any convenient image analysis program.

Human THP-1 cells. The human acute monocytic leukemia cell line THP-1 used in this study was maintained at 37°C in a humidified incubator containing 5% CO₂. These cells were grown in RPMI-1640 medium (Gibco BRL) supplemented with 10% fetal calf serum (Gibco BRL), glucose (4.5 g/l), 5 x 10⁻⁵ M b-mercaptoethanol, 1 mM sodium pyruvate, streptomycin (100mg/ml), and penicillin (100 U/ml). The cells were split into 6-well cell culture plates, 5 ml per plate, at a density of 5 x 10⁵ cells/ml in complete RPMI1640 medium. Before activation of the cells with *E. coli* or *P. aeruginosa* as inflammantory stimuli, the cells were treated with 1.6 x 10⁻⁹ M phorbol-12-myristate-13 acetate (PMA) (Sigma) for 48hr. After this incubation time the cells were treated with different concentrations (1 x 10¹-10⁵ cells/ml) of exponentially grown *E. coli* and *P. aeruginosa* bacterial cells for 8hr. Both supernatant fluids and the cell pellet were collected and stored at -80 °C for later cytokine assays and RNA isolation / RT-PCR dectection of cytokine mRNA.

Isolation of RNA. The RNA isolation method with TRIzol Reagent (Gibco BRL) used is essentially as developed by Chomoczynski, P., and N. Sacchi.(1987, Anal. Biochem. 162:156-159). The frozen cell pellets (about 2.5 x 10⁶ cells) were lysed by resuspending in 1 ml of TRIzol Reagent (Gibco BRL) and repetitive pipetting. 200 ml of chloroform was than added and the samples were mixed vigorously by vortexing for15 seconds. After centrifugation of the samples at 14,000 rpm for 15 min at 4 °C, the colorless upper aqueous phase containing the RNA was transferred into a fresh RNase-free tube. The RNA was precipitated from the aqueous phase by mixing with 0.5 ml of isopropyl alcohol. Samples were incubated at room temperature for 10 min before centrifugation at 4°C for 7,500 rpm for 10 min. The RNA pellet was washed once with 0.5 ml of 75% ethanol, before allowed to air dry for 10 min. The concentration and purity of the RNA isolated was determined by measuring the OD₂₆₀ in 2 mM Na₂PO₄ (pH8.0) in a spectrophotometer and by agarose gel electrophoresis.

RT-PCR detection of cytokine mRNA. The RT-PCR assay used in this work is essentially as described elsewhere (Murphy *et al*., 1993, J. Immunol. Methods. 162:211-223). Oligonucleotide primers targeting a 206-bp region of the human IL-6 gene (Zilberstein *et al*., 1986, EMBO J. 5:2529-37) were used to detect IL-6 specific mRNA in treated THP-1 cells. These IL-6 specific PCR primers were designed based on the NCBI sequence query for the human IL-6 gene (accession number X04430). Oligonucleotide primers targeting a 107-bp region of the human IL-1β gene (Nishida *et al*., 1987) were used to detect IL-1β specific mRNA in untreated and treated THP-1 cells. These IL-1β specific PCR primers were designed based on the NCBI sequence query for the human IL-1β gene (accession number M15330). All the primers were prepared by Operon (Alameda, CA). RT (reverse transcriptase) reactions were carried out in a final volume of 20 ml containing 1 mg of total RNA and the components supplied by PE Applied Biosystems (Foster City, CA) in the TaqMan Gold RT-PCR kit. RT reactions were performed as described in the kit protocol. For the following PCR reaction 1 ml of the RT reaction sample was used in a final volume of 30 ml containing all the components supplied by PE Applied Biosystems in the TaqMan Gold RT-PCR kit. PCR reactions were performed as described in the kit protocol. 10 ml of each PCR product was loaded onto a 3% NuSieve/1%SeaKem GTG Agarose gel (FMC BioProducts, Rockland, ME) in 1xTBE buffer and subsequent electrophoresis was performed at 200V for 30 - 45 min.

## Claims

1. A solid phase array comprising at least five different antibodies arrayed in corresponding discrete array elements in fluid connection and specific for corresponding five different cytokines, wherein each antibody is specific for and bound to a corresponding sample cytokine, to permit quantitative detection of the sample cytokines.

2. An array according to claim 1, comprising at least ten different antibodies arrayed in corresponding discrete array elements and specific for corresponding ten different cytokines, wherein each antibody is specific for and quantitatively bound to a corresponding sample cytokine.

3. An array according to claim 1 or 2, wherein the cytokines are selected from Table 1.

4. An array according to claim 1 or 2, wherein the cytokines are selected from: GM-CSF, IL1α, IL1β, IL2, IL4, IL6, IL7, IL8, IL10, IL12 and TNF α.

5. An array according to claim 1, 2, 3 or 4, wherein the antibodies are monoclonal.

6. A method for the simultaneous, quantitative detection of cytokines in a sample, the method comprising the step of contacting the sample with a solid phase array comprising at least five different antibodies arrayed in corresponding discrete array elements in fluid connection and specific for corresponding five different cytokines, whereby at least five different corresponding sample cytokines are simultaneously quantitatively measured.

7. A method according to claim 6, wherein the array comprises at least ten different antibodies arrayed in corresponding discrete array elements and specific for corresponding ten different cytokines, whereby at least ten different corresponding sample cytokines are simultaneously quantitatively measured.

8. A method according to claim 6 or 7, wherein the cytokines are selected from Table 1.

9. A method according to claim 6 or 7, wherein the cytokines are selected from: GM-CSF, IL1α, IL1β, IL2, IL4, IL6, IL7, IL8, IL10, IL12 and TNFα.

10. A method according to claim 6, 7 or 8, wherein the antibodies are monoclonal.

## Patentansprüche

1. Eine Festphasenanordnung, umfassend wenigstens fünf verschiedene Antikörper, die in entsprechenden diskreten Anordnungselementen in fluider Verbindung angeordnet sind und für entsprechende fünf verschiedene Cytokine spezifisch sind, worin jeder Antikörper für ein entsprechendes Probencytokin spezifisch und daran gebunden ist, um eine quantitative Detektion der Probencytokine zu ermöglichen.

2. Eine Anordnung gemäß Anspruch 1, umfassend wenigstens zehn verschiedene Antikörper, die in entsprechenden diskreten Anordnungselementen angeordnet sind und für entsprechende zehn verschiedene Cytokine spezifisch sind, wobei jeder Antikörper für ein entsprechendes Probencytokin spezifisch und quantitativ daran gebunden ist.

3. Eine Anordnung gemäß Anspruch 1 oder 2, worin die Cytokine aus der Tabelle 1 ausgewählt sind.

4. Eine Anordnung gemäß Anspruch 1 oder 2, worin die Cytokine ausgewählt sind aus: GM-CSF, IL1 α, IL1 β, IL2, IL4, IL6, IL7, IL8, IL10, IL12 und TNF α.

5. Eine Anordnung gemäß einem der Ansprüche 1, 2, 3 oder 4, worin die Antikörper monoklonal sind.

6. Ein Verfahren zur simultanen, quantitativen Detektion von Cytokinen in einer Probe, wobei das Verfahren den Schritt des Inkontaktbringens der Probe mit einer Festphasenanordnung umfasst, die wenigstens fünf verschiedene Antikörper umfasst, die in entsprechenden diskreten Anordnungselementen in fluider Verbindung angeordnet sind und für entsprechende fünf verschiedene Cytokine spezifisch sind, wobei wenigstens fünf verschiedene entsprechende Probencytokine simultan quantitativ gemessen werden.

7. Ein Verfahren gemäß Anspruch 6, worin die Anordnung wenigstens zehn verschiedene Antikörper umfasst, die in entsprechenden diskreten Anordnungselementen angeordnet sind und für entsprechende zehn verschiedene Cytokine spezifisch sind, wobei wenigstens zehn verschiedene entsprechende Probencytokine simultan quantitativ gemessen werden.

8. Ein Verfahren gemäß Anspruch 6 oder 7, worin die Cytokine aus der Tabelle 1 ausgewählt sind.

9. Ein Verfahren gemäß Anspruch 6 oder 7, worin die Cytokine ausgewählt sind aus: GM-CSF, IL1 α, IL1 β, IL2, IL4, IL6, IL7, IL8, IL10, IL12 und TNF α.

10. Ein Verfahren gemäß einem der Ansprüche 6, 7 oder 8, worin die Antikörper monoklonal sind.

## Revendications

1. Arrangement en phase solide comprenant au moins cinq anticorps différents arrangés dans des éléments correspondants en connexion fluide et spécifique de cinq cytokines différentes correspondantes, dans lesquels chaque anticorps est spécifique et se fixe à un échantillon de cytokines correspondant pour permettre une détection quantitative des cytokines dans l'échantillon.

2. Arrangement selon la revendication 1, comprenant au moins dix anticorps différents arrangés dans des éléments correspondants et spécifiques de dix cytokines différentes, chaque anticorps étant spécifique et fixé de façon quantitative à une cytokine d'un échantillon.

3. Arrangement selon la revendication 1 ou 2, dans lequel les cytokines sont choisies dans le Tableau 1.

4. Arrangement selon la revendication 1 ou 2, dans lequel les cytokines sont choisies parmi le GM-CSF, l'IL1α, l'IL1β, l'IL2, l'IL4, l'IL6, l'IL7, l'IL8, l'IL10, l'IL12 et le TNFα.

5. Arrangement selon l'une des revendications 1 à 4, dans lequel les anticorps sont des anticorps monoclonaux.

6. Procédé de détection simultanée quantitative de cytokines dans un échantillon, comprenant les étapes de mettre en contact l'échantillon avec un arrangement en phase solide comprenant au moins cinq anticorps différents arrangés dans des éléments correspondants en connexion fluide et spécifiques de cinq cytokines différentes, dans lequel au moins cinq cytokines différentes de l'échantillon sont mesurées de façon simultanée et quantitativement.

7. Procédé selon la revendication 6, dans lequel l'arrangement comprend au moins dix anticorps différents arrangés dans des éléments correspondants et spécifiques de dix cytokines différentes, dans lequel au moins dix cytokines différentes sont mesurées simultanément et quantitativement.

8. Procédé selon l'une des revendications 6 ou 7, dans lequel les cytokines sont choisies dans le Tableau 1.

9. Procédé selon l'une des revendications 6 ou 7, dans lequel les cytokines sont choisies parmi le GM-CSF, l'IL1α, l'IL1β, l'IL2, l'IL4, l'IL6, l'IL7, l'IL8, l'IL10, l'IL12 et le TNFα.

10. Procédé selon l'une quelconque des revendications 6, 7 ou 8 dans lequel les anticorps sont des anticorps monoclonaux.
